# EUROPEAN PATENT APPLICATION

(11) **EP 2 698 133 A1**
(43) Date of publication of application: **19.02.2014**
(21) Application number: 12771290.9
(22) Date of filing: 11.04.2012
(51) Int. Cl.: A61F 7/08

(54) **HEAT GENERATOR**

(30) Priority: 12.04.2011 JP 2011087950; 08.02.2012 JP 2012025044
(71) Applicant: Mycoal Co., Ltd., Tochigi-shi, Tochigi 328-0067 (JP)
(72) Inventor: USUI, Yasumasa, Tochigi-shi, Tochigi 328-0067 (JP)
(74) Representative: Körfer, Thomas
(86) International application number: PCT/JP2012/002500
(87) International publication number: WO 2012/140875

(57) **Abstract**

An object of the present invention is to provide a heat generating body giving no uncomfortable feeling when in use and causing no direct close contact between a heat generating member and the skin by an adhesive to relieve the burden of the skin while eliminating a low temperature burn even for a person with a delicate skin and in the case of continuous use. The heat generating body of the present invention is characterized in that the heat generating member having a wide surface is provided on a mesh-like sheet larger in outer shape than the one-side wide surface of the heat generating member and that an adhesive layer is provided on the mesh-like sheet located at the outer peripheral part of the heat generating member.

## Description

### Technical Field

The present invention relates to a heat generating body that has a heat generating member composed to generate heat by a heat generating composition such as iron powder and that is used directly stuck to the skin of the body.

### Background Art

The conventionally known heat generating body of this kind is, as shown in Patent Document 1, used stuck to the skin utilizing an adhesive layer formed on the rear face, which is the sticking side to the body, of a heat generating member.

However, this conventional heat generating body is used stuck to the skin through the adhesive layer formed on the rear face, it has a problem of giving an uncomfortable feeling when in use because the heat generating body formed with the adhesive layer is not deformed following the movement of the body. In the case of using the heat generating body for a long time, there have been also a problem of causing a low temperature burn because the adhesive layer of the heat generating member continues to be in close contact with the skin of the body, and inconvenience of stripping off the skin in the case of trying to strip off the heat generating body together with the adhesive layer when causing the low temperature burn.

### Citation List

### Patent Document

Patent Document 1: JP-A-05-208031

### Summary of the Invention

### Problems to be Solved by the Invention

An object of the present invention is to provide a heat generating body giving no uncomfortable feeling when in use and causing no direct close contact between the heat generating member and the skin by an adhesive to relieve the burden of the skin while eliminating a low temperature burn even for a person with a delicate skin and in the case of continuous use.

### Means of Solving the Problems

In order to solve the above problem, the present inventor found, as a result of thorough examination, that the adhesive layer can be eliminated from the heat generating member portion not by providing the heat generating member with the adhesive layer to stick the heat generating member to the skin through the adhesive layer as in the conventional art, but by providing the heat generating member on the sticking side of a mesh-like sheet larger in outer shape than the heat generating member, and sticking the heat generating member to the skin through an adhesive layer provided at the mesh-like sheet extending to the outer periphery of the heat generating member. The heat generating member is not thereby brought into direct close contact with the skin to eliminate the uncomfortable feeling when in use. Further, the slight movement of a user easily causes in-and-out of air between the heat generating member and the skin to maintain the moderate sense of warmth. As a result, the burden on the skin is relieved for a person with a delicate skin and in the case of continuous use, and the low temperature burn can be prevented.

A heat generating body which is a first solution means of the present invention is characterized in that a heat generating member having a wide surface is provided on a mesh-like sheet larger in outer shape than the one-side wide surface of the heat generating member and that an adhesive layer is provided on the mesh-like sheet located at the outer peripheral part of the heat generating member.

A second solution means is characterized in that the mesh-like sheet is provided with holes.

A third solution means is characterized in that the adhesive layer is formed on the whole one-side surface of the mesh-like sheet and that the heat generating member is stuck to the mesh-like sheet through the adhesive layer.

A fourth solution means is characterized in that the adhesive layer is formed in a range of 10 to 80% of the surface area of a region, in which the heat generating member is located, on the mesh-like sheet (excluding a seal part when the seal part is formed at the peripheral edge of the wide surface of the heat generating member), and the adhesive layer is formed at the seal part of the heat generating member extending in an orthogonal direction to the longitudinal direction of the heat generating body.

A fifth solution means is characterized in that, in the case where the heat generating body has a plurality of heat generating members, a region surrounded by the heat generating members located on the outermost peripheral edge is made the above-mentioned region (excluding the seal parts of the heat generating members located at both ends in the longitudinal direction of the heat generating body in the above-mentioned region).

A sixth solution means is characterized in that non-adhesive sections without the adhesive layer in the above-mentioned region are connectedly formed also on the mesh-like sheet while adjusting the width of the non-adhesive sections in a direction orthogonal to the longitudinal direction of the heat generating body.

A seventh solution means is characterized in that the adhesive strength of the adhesive layer is 2.0 to 3.3 N/10.5 mm and that the shearing force is 6.0 to 8.5 kgf/29 mm.

An eighth solution means is characterized in that the heat generating member is formed by holding a heat generating composition between a covering material formed of an air permeable material located on the mesh-like sheet side, and a base material formed of an air impermeable material located on the side opposite to the mesh-like sheet, and enclosing the heat generating composition between the covering material and the base material so as to form a seal part at the outer peripheral edges of the covering material and base material.

A ninth solution means is characterized in that the contact surface side of the heat generating member is covered with a protective sheet.

A tenth solution means is characterized in that the protective sheet is a non-woven fabric.

An eleventh solution means is characterized in that a resin film is provided on the heat generating member side of the protective sheet.

A twelfth solution means is characterized in that protrusions with a height of 0.3 to 2.0 mm are provided on the contact surface side of the protective sheet.

A thirteenth solution means is characterized in that a release sheet is provided at a part or the whole of the adhesive layer.

A fourteenth solution means is characterized in that a medicine is contained in a part of at least one member out of the surface of the heat generating member, the mesh-like sheet and the protective sheet which are components of the heat generating body.

A fifteenth solution means is characterized in that the medicine is contained in the heat generating body by arranging a medicine layer adjacently to the heat generating body.

A sixteenth solution means is characterized in that the mesh-like sheet is a non-woven fabric.

A seventeenth solution means is characterized in that as to the elongation percentage difference in the longitudinal direction of the heat generating body between the mesh-like sheet and the heat generating member provided at the heat generating body, the mesh-like sheet is larger by 10% or more.

An eighteenth solution means is characterized in that mesh-like sheets are provided at both ends of the wide surface of the heat generating member having the wide surface and that an adhesive layer is provided on each mesh-like sheet.

### Effects of the Invention

According to the present invention, the heat generating member of flat shape, sheet-shape, or the like with the wide surface is provided on one face side which is the sticking side of the mesh-like sheet larger than the outer shape of the wide surface of the heat generating member, and an adhesive layer is provided on the mesh-like sheet located on the outer peripheral part of the heat generating member. The heat generating body can thereby be stuck to the skin of the body through the adhesive layer provided on the rear face of the mesh-like sheet extending to the outer periphery of the heat generating member. Since the heat generating member itself is not stuck to the body, even if the mesh-like sheet is deformed following the movement of the body, independence is kept without completely following this deformation to eliminate an uncomfortable feeling when in use. Further, since the heat generating member is not in close contact with the skin through a full-face adhesive, the slight deformation of the mesh-like sheet easily causes in-and-out of air between the heat generating member and the skin to maintain the moderate sense of warmth. As a result, the burden on the skin is relieved when used by a person with a delicate skin or used continuously for many hours, and the low temperature burn can be prevented.

An air permeability rate and sticking force can be adjusted by providing the mesh-like sheet with holes larger than the average hole diameter of holes provided in the mesh-like sheet. If the holes themselves provided in the mesh-like sheet are uniformly provided, the air permeability rate and sticking force become uniform, but the air permeability rate and sticking force can be adjusted by the optionally selected hole size. On the other hand, if the hole diameters are made different between the longitudinal center part and both ends or the like of the heat generating body, the temperature of the heat generating body can be uniformed as a whole, for instance. Further the sticking force, applied to the skin of the body, of the adhesive layer provided on the rear face of the mesh-like sheet located at the outer peripheral part of the heat generating member can be suitably adjusted.

The size of the heat generating member can be suitably changed by forming the adhesive layer on the whole rear face of the mesh-like sheet and sticking the heat generating member to the mesh-like sheet through the adhesive layer. The heat generating body can thereby be made corresponding to an applied part of the body.

The adhesive layer is formed within a range of 10 to 80% of the surface area of a region, in which the heat generating member is located, on the mesh-like sheet (excluding a seal part if the seal part is formed at the peripheral edge of the wide surface of the heat generating member), and adhesive layers of the predetermined width are formed at predetermined spaces at the seal part of the heat generating member extending in the orthogonal direction to the longitudinal direction of the heat generating body. An air permeability rate enough to obtain the sufficient sense of warmth as the heat generating body can thereby be obtained, and simultaneously the heat generating member can be firmly stuck to the mesh-like sheet so as not to fall off. The region may be based on heat generation characteristics requested at the body applied part or the like of the heat generating body and does not necessarily need to have a high or low air permeability rate. However, since the adhesive layers of the predetermined area ratio are provided, sticking between the mesh-like sheet and the heat generating member can be made firm according to the magnitude of movement of the applied part.

In the case of the heat generating body having a plurality of heat generating members at least in the longitudinal direction of the heat generating body or also in the orthogonal direction to the longitudinal direction, the region surrounded by the heat generating members located at the outermost peripheral edge is made the region, in which the heat generating members are located, on the mesh-like sheet (excluding the seal parts of the heat generating members located at both ends in the longitudinal direction of the heat generating body in the region). The heat generating body can thereby be conformed to the shape of the applied part of the body, and further as the heat generating member, the heat generating body of equal temperature on the whole can be obtained.

In the region, in which the heat generating member is located, on the mesh-like sheet, the non-adhesive sections without the adhesive layer are connectedly formed also on the mesh-like sheet while adjusting the width of the non-adhesive sections in the direction orthogonal to the longitudinal direction of the heat generating body. One length of the adhesive provided particularly at the long side of the orthogonal direction to the longitudinal direction of the heat generating body can be shortened, and when the heat generating body is stuck to the skin of the body, a force caused by the movement to deform the heat generating body is dispersed to obtain the heat generating body that relieves an uncomfortable feeling.

Further, since the adhesive strength of the adhesive layer is set to 2.0 to 3.3 N/10.5 mm, and the shearing force is set to 6.0 to 8.5 kgf/29 mm, the heat generating body hardly falls off the stuck body (skin) while relieving the uncomfortable feeling such as a stiff feeling during use, and the heat generating body can be stripped off without giving a sharp pain or the like to the user after use.

The heat generating member is formed by holding the heat generating composition between the covering material formed of the air permeable material located on the mesh-like sheet side, and the base material formed of the air impermeable material located on the side opposite to the mesh-like sheet, and enclosing the heat generating composition between the covering material and the base material so as to form the seal part at the outer peripheral edges of the covering material and base material. Heat generation for many hours at a stable temperature is thereby attained.

In the case of covering the contact surface side of the heat generating member with the protective sheet, the direct contact of the heat generating member with the skin can be prevented to eliminate a risk of a low temperature burn.

In the case of forming the protective sheet of a nonwoven fabric, cost is low, and the surface touch of the nonwoven fabric gives a pleasant feeling of contact with the skin. Further, the sense of warmth can be adjusted by the user himself or herself by laminating a plurality of protective sheets for use.

In the case of providing the resin film on the heat generating member side of the protective sheet, the temperature change of the heat generating body when the user moves to a place with a different use time environmental temperature can be made comparatively small.

In the case of providing the protrusions with a height of 0.3 to 2.0 mm on the contact surface side of the protective sheet, an air layer is formed to cause a heat insulating effect. The sense of warmth can thereby be adjusted even with the thin protective sheet, and since unevenness can be provided in a manufacturing process, cost is reducible, and the feel in contact with the skin can be pleasant.

In the case of providing the release sheet at a part or the whole of the adhesive layer of the mesh-like sheet, the adhesive layer can be protected until the time of use, and a fall in the adhesive strength of the adhesive layer can be prevented.

In the case of containing a medicine in a part of at least one member out of the surface of the heat generating member, the mesh-like sheet and the protective sheet which are components of the heat generating body, a synergistic effect of a medicinal effect combined with a heating effect with no uncomfortable feeling can be expected.

In the case of containing a medicine in the heat generating body by arranging the medicine layer adjacently to the heat generating body, the synergistic effect of the medicinal effect combined with the heating effect with no uncomfortable feeling can be surely expected for many hours.

The mesh-like sheet is formed of the nonwoven fabric having an air permeability rate, extensibility or expansibility, and high strength. The heat generating body low in cost can thereby be obtained.

As to the elongation percentage difference in the longitudinal direction of the heat generating body between the mesh-like sheet and the heat generating member provided at the heat generating body, the mesh-like sheet is larger by 10% or more. The uncomfortable feeling such as the stiff feeling caused by the movement of the user's body hardly occurs and can be relieved, and since the shearing force is made slightly large, the heat generating body hardly falls off the body (the skin).

According to the present invention, the mesh-like sheets are provided at both ends of the wide surface of the heat generating member of flat shape, sheet-shape, or the like having the wide surface, and the adhesive layer is provided on each mesh-like sheet. The burden on the user's skin can thereby be reduced, and flexibility is produced as the whole heat generating body. Consequently, the heat generating body giving a more fit sensation and relieving the uncomfortable feeling when applied to a large curve part of the body can be obtained.

### Brief Description of the Drawings

Fig. 1 is an explanatory view of a heat generating body of one embodiment in the present invention.
Fig. 2 is an explanatory view of a heat generating body of another embodiment in the present invention.
Fig. 3 is an explanatory view showing the relationship between a heat generating member and a mesh-like sheet of a heat generating body in the present invention.
Fig. 4 is an explanatory view of a heat generating body provided with holes in a mesh-like sheet in the present invention.
Fig. 5 is an explanatory view of an adhesive layer of a heat generating body in the present invention.
Fig. 6 is an explanatory view of an adhesive layer of a heat generating body of another embodiment in the present invention.
Fig. 7 is a photograph showing an example of the present invention provided with a release sheet.
Fig. 8 is a photograph showing an example provided with another release sheet instead of the above release sheet.
Fig. 9 is a graph showing heat generation characteristics in Examples 1 to 2 of the present invention.
Fig. 10 is a graph showing temperature measurement results in Examples 3a to 3c.
Fig. 11 is a graph showing temperature measurement results in Examples 4a and 4b.
Fig. 12 is a graph showing temperature measurement results in Examples 5a to 5c.
Fig. 13 is a graph showing temperature measurement results in Examples 6a to 6c.
Fig. 14 is a graph showing temperature measurement results in Examples 9a to 9c.
Fig. 15 is a graph showing a temperature measurement result in Example 10a.
Fig. 16 is a graph showing a temperature measurement result in Example 10b.
Fig. 17 is a graph showing a temperature measurement result in Example 10c.

### Mode for Carrying Out the Invention

Embodiments of the present invention are explained referring to drawings as follows.

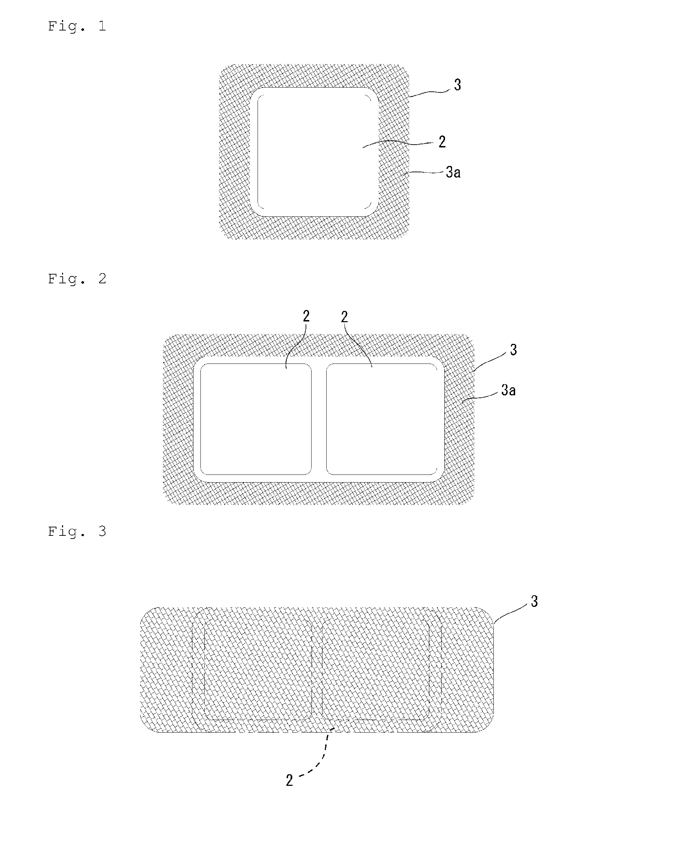

A heat generating body 1 shown in Fig. 1 is formed by providing a heat generating member 2 having, on its front and rear sides, wide surfaces with respect to thickness, on one-side surface of a mesh-like sheet 3 larger than the outside dimensions of the wide surface of the heat generating member 2. An adhesive layer 3a is provided on one surface side of the mesh-like sheet 3 extending to the outer periphery of the heat generating member 2.

The heat generating member 2 itself is not directly stuck to the body. Independence is thereby kept in comparison with the case of the mesh-like sheet being deformed following the movement of the body, and there is no uncomfortable feeling when in use. Further, there is no adhesive layer on the contact surface of the heat generating member 2, and the heat generating member 2 does not directly come in close contact with the skin. Consequently, the slight movement of the user easily causes in-and-out of air between the heat generating member 2 and the skin through the mesh-like sheet 3 to maintain the moderate sense of warmth. As a result, the burden on the skin is relieved for a person with a delicate skin or in the case of continuous use, and the low temperature burn can be prevented.

A heat generating body 1 shown in Fig. 2 is a deformed example of the heat generating body 1 in Fig. 1. Heat generating members 2 integrally formed as an assembly of two heat generating members provided beforehand at a space are provided on the one-side surface of a mesh-like sheet 3.

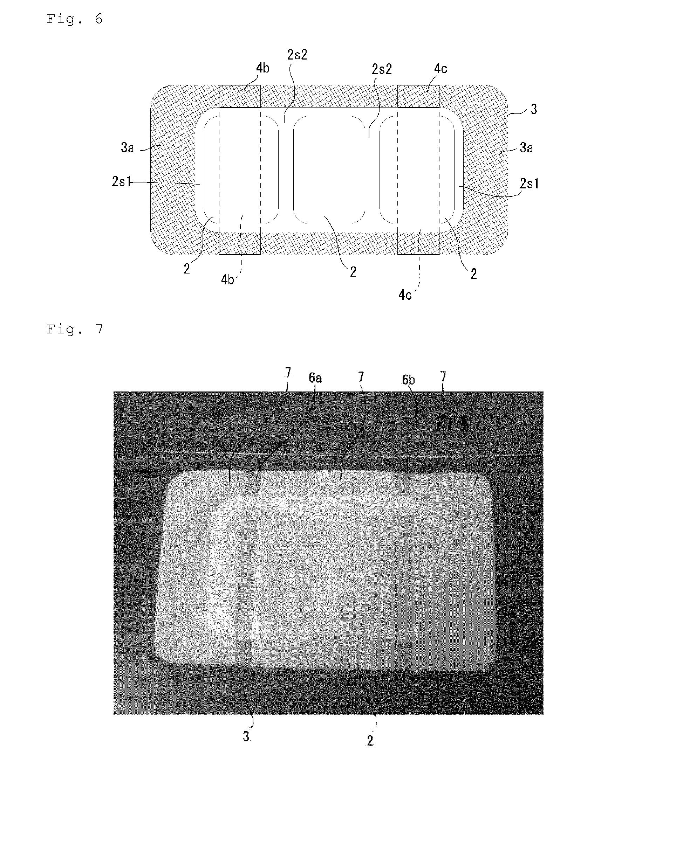

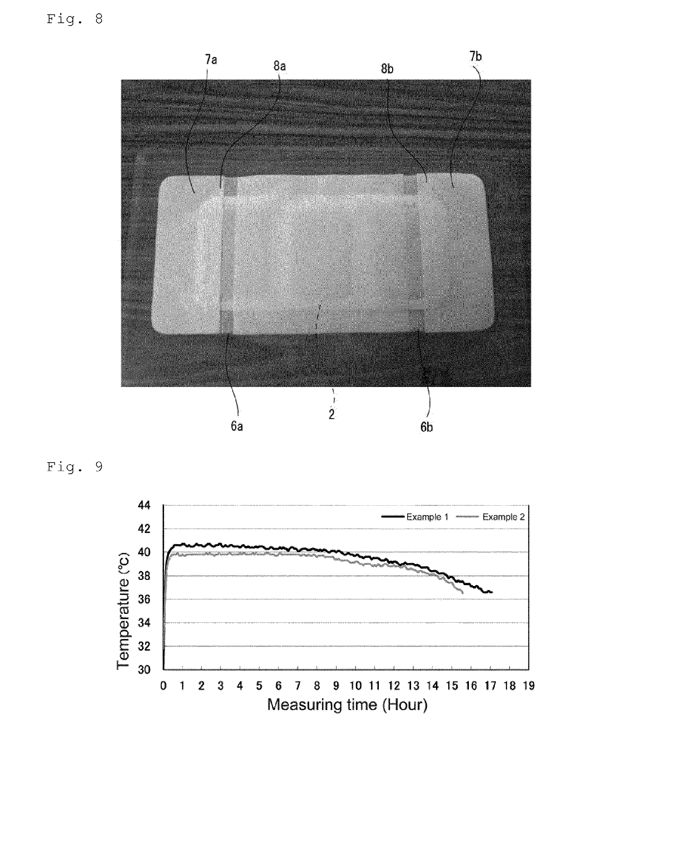

The heat generating members 2 may be integrally formed as an assembly of three or more heat generating members provided beforehand at spaces and arranged in the longitudinal direction of a long-sized mesh-like sheet 3 so that the heat generating members 2 are uniform in the longitudinal direction as shown in Fig. 6 and Fig. 8. Alternatively, the heat generating members 2 may be arranged in plane not only in one direction but also in two directions or the like of a matrix although not shown in the figure.

Modes of providing the heat generating members 2 with respect to the mesh-like sheet 3 include a mode of making the length of the heat generating members 2 and mesh-like sheet 3 uniform in one direction as shown in Fig. 3 or a mode of providing the mesh-like sheets 3 only at both ends of the heat generating members 2 although not shown in the figure.

The size of the heat generating body 1 is not limited as long as an effect can be expected. However, in the case of the heat generating body 1 of square plane shape as shown in Fig. 1, the length and breadth of the heat generating body 1 are set to 50 to 200 mm respectively, and the length and breadth of the heat generating member 2 may be set to 30 to 150 mm respectively. In the case of the long-sized heat generating body 1 as shown in Fig. 2, the heat generating body 1 may be 50 to 200 mm long and 75 to 300 mm broad, and the whole of the heat generating members 2 may be 30 to 150 mm long and 50 to 250 mm broad.

In the case where one heat generating member is rectangular, it is preferable to arrange the one heat generating member so that its long side is adjusted to a direction orthogonal to the longitudinal direction of the heat generating body 1 as shown in Fig. 6 and Fig. 8.

The breadth of the mesh-like sheet 3 located at the outer peripheral part of the heat generating member 2 is not limited as long as it can be stuck to the skin of the body but can be optionally selected according to the size, weight, etc. of the heat generating member 2. In the case of using a chemical body warmer or the like normally on the market as the heat generating member 2, the breadth may be made 5 to 30 mm.

The corners of the mesh-like sheet 3 and heat generating member 2 may be provided with optional roundness (R: radius) if necessary in order to ease and reduce an uncomfortable feeling in contact with the skin when sticking the heat generating body to the skin of the body, and the roundness is preferably R5 (mm) or more.

The "mesh-like sheet" in the present invention is to have air permeability as well as follow-up property to the skin to the extent of not impairing the function of the stuck heat generating body when the mesh-like sheet is used stuck to the skin of the body in the same way as a clothes-sticking type chemical body warmer or the like.

Specifically, the "mesh-like sheet" is a sheet deformable following the movement of the body, more specifically, a sheet having air permeability as well as expansibility or extensibility in both length and breadth directions, or a sheet having air permeability as well as expansibility or extensibility in one direction out of the length and breadth directions without having expansibility or extensibility in the remaining direction.

Material is not particularly limited, and examples can be an air permeable film such as a porous film or a perforated film; an independently air-permeable material such as paper or a nonwoven fabric; an air permeable material with at least one kind of an air permeable film, a nonwoven fabric, etc. laminated on paper; an air permeable material obtained by perforating an air impermeable wrapping material with a polyethylene film laminated on a nonwoven fabric; a nonwoven fabric or a porous film with fiber laminated, thermocompressed and controlled in air permeability, or a laminate of the porous film and the nonwoven fabric. The perforated film is provided with air permeability by providing pores in an air impermeable film such as a polyethylene film with a needle.

The mesh-like sheet may be provided with holes (openings) large compared with the average pore diameter of the above pores provided for the purpose of aeration. Even if an adhesive is solidly applied to the whole surface of the mesh-like sheet, air permeability required for heat generation can be obtained, and a required sticking force can be obtained. The shape, size, number, etc. of the holes (openings) are not particularly limited as long as air permeability required for the heat generation of the heat generating body of the present invention and sticking force (moderate adhesive strength and shearing force) required to stick to the skin can be obtained.

The shape of the holes (openings) may be a round shape such as a circular or elliptical shape, or a polygonal shape such as a triangular or quadrilateral shape, for example. The maximum outside dimensions of the holes (openings) may be about 0.1 to 100 mm.

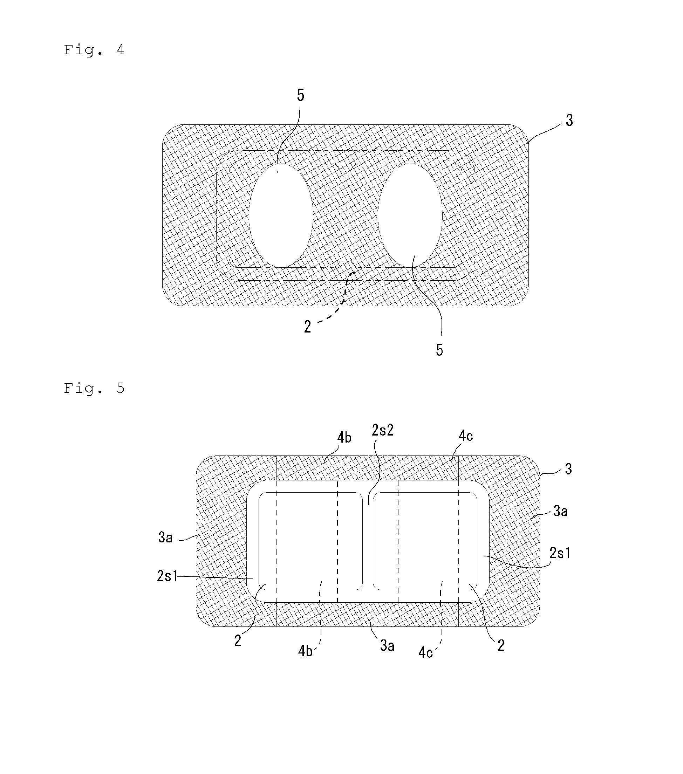

An explanation on the holes (openings) is made referring to Fig. 4, which is an example of providing the mesh-like sheet 3 provided with the holes (openings) 5, 5 in the corresponding (overlapping) position of the heat generating member 2 from the opposite side to the sticking surface of the heat generating body 1 provided with the heat generating member 2.

Regarding the air permeability of the mesh-like sheet, there are no particular restrictions as long as heat generation can be maintained.

It is preferable to use a sheet obtained by laminating an air permeable film on the mesh-like sheet, as a covering material for covering a below-mentioned heat generating composition to form the heat generating body because the manufacture of the heat generating body of the present invention is facilitated. With respect to the air permeability of the thus laminated sheet, in the case of forming the heat generating body using the below-mentioned heat generating composition, the sheet with a moisture permeability of about 50 to 10,000 g/m²/24 hr. measured by the Lissy method can be used. If the moisture permeability is less than 50 g/m²/24 hr., the amount of heat generation is reduced, and a sufficient heating effect cannot be obtained. On the other hand, if the moisture permeability exceeds 10,000 g/m²/24 hr., the heat generating temperature becomes high, and there is a risk of causing a safety problem. Therefore neither is preferable.

In the case of not laminating the mesh-like sheet and the covering material unlike the above-mentioned sheet, it is preferable to set the mutual air permeability rate ratio of the air permeability rates thereof such that if the air permeability rate of the covering material is 1, that of the mesh-like sheet is 0.8 or more, more preferably 1.0 or more, even more preferably 1.2 or more. If the air permeability rate of the mesh-like sheet is less than 0.8, it is not preferable because there is a risk of causing insufficient heat generation. With respect to the air permeability of each of the mesh-like sheet and the covering material, an air permeability rate measured by an air permeability rate measuring apparatus can be about 1.0 to 21.0 ml/min/506.7 mm². If the air permeability rate is less than 1.0 ml/min/506.7 mm², the amount of heat generation is reduced, and a sufficient heating effect cannot be obtained. On the other hand, if the air permeability rate exceeds 21.0 ml/min/506.7 mm², the heat generating temperature becomes high, and there is a risk of causing a safety problem. Therefore neither is preferable.

The "air permeability rate" in this description is measured based on a testing method JIS-L1096. The longitudinal-lateral dimensions of a sample is set to 100 x 100 mm or more, and it is set such that Φ75±5 mm which is an area to be measured can be measured. The sample is then left for 60 minutes in a thermostatic chamber (20±3°C). The sample is set on a measuring part (a lower part) provided with a plate ring-shaped packing on the upper face of an air permeability rate measuring apparatus installed in the same conditions such that the laminate film side (the side of a porous film or the like) of the sample is the top. At the start of measurement, the measuring part (an upper part) provided with an O-ring-shaped packing at the lower face descends, and the sample is clamped between the measuring part (the lower part) and the measuring part (the upper part). An air pressure of 0.1 kg/cm² is applied to the sample from the measuring part (the upper part) to the measuring part (the lower part) side, and after about 20 seconds, a numerical value converted from the amount of air permeating the sample (in a stable state) is set as an air permeability rate (ml/min/506.7 mm²). This 506.7 mm² is per area of a circle of Φ1 inch. In measuring the air permeability rate, an air permeability rate measuring apparatus (made by Asia Create Co., Ltd.) is used for a testing apparatus installed in the thermostatic chamber (20±3°C), and after power-on, warming up is performed until the apparatus is stabilized (about 15 minutes). Afterwards, as measurement preparations, the air pressure applied to the sample is set to 0.1 kg/cm², and the display of 0 on a zero point measuring sheet (a PET film of about 100 µm is confirmed (a zero point adjustment is made if necessary), and the display of a predetermined reference point by a standard plate (a measuring plate specified by an air permeability rate measuring apparatus maker) is confirmed (a reference point adjustment is made if necessary). No stain or the like on the packing is confirmed, and if there is a stain, the stain is removed by rubbing it with a dry cotton cloth or the like to set a state in which the air permeability rate of the sample can be measured.

The extension percentage of the mesh-like sheet in a TD direction (a longitudinal direction in which the heat generating members are continuous in the heat generating body provided with a plurality of heat generating members at spaces) is preferably larger by 10% or more than the longitudinal extension percentage of the heat generating member. The occurrence of an uncomfortable feeling such as a stiff feeling caused by the movement of the body can be further suppressed and relieved by having expansibility or extensibility in the TD direction.

Specifically, the extension percentage of the mesh-like sheet in the TD direction is 10% or more, preferably 30% or more, further preferably 50% or more to provide a difference from the extension percentage of the heat generating member. If the extension percentage of the mesh-like sheet in the TD direction is less than 10%, the stiff feeling occurs due to the movement of the body to cause the fall of the heat generating body or to cause the uncomfortable feeling of pulling the skin, which is unpleasant. The extension percentage of the mesh-like sheet in an MD direction is not particularly restricted but is preferably less than 10% from the viewpoint of manufacturing with high accuracy.

The strength of the mesh-like sheet in the TD direction is set to 3 N/25 mm or more, preferably to 5 N/25 mm or more, further preferably to 10 N/25 mm or more. If the strength is less than 3 N/25 mm, the strength is low, and the mesh-like sheet cannot cope with the movement of the body so as to be easy to break.

With respect to the "extension percentage" in this description, the strength of a base material is first measured according to a testing method of JIS-Z1707. The mesh-like sheet is formed in strip shape with width 25±0.5 mm x length 150±1.0 mm to form a test piece, which is left for 60 minutes in a thermostatic chamber (20±3°C). The initial distance between chucks of a testing apparatus is set to 100±1.0 mm, and the tensile moving speed is set to 500±10 mm/min, that is, the test piece is prepared according to JIS-K7127/2/500. The extension percentage is computed from the following expression after measuring elongation (mm) at the display of the base material strength (a maximum load):
Extension percentage (%)=(elongation measured value (mm)/test piece length (mm)) x 100, wherein the test piece length when computing the extension percentage is the initial distance 100±1.0 mm between the chucks of the testing apparatus.

In measuring the base material strength, a tension testing machine with a thermostatic bath (made by TESTER SANGYO CO., LTD.) is used for the testing apparatus.

An adhesive used in the present invention is not limited as long as it has adhesive strength required to stick to the skin, and a solvent-based, water-based, emulsion-based, hot-melt, reactive, pressure-sensitive or non-hydrophilic adhesive, or a hydrophilic adhesive may be used. Since the heat generating body of the present invention is used directly stuck to the skin of the body, it is preferable to use the water-based adhesive without skin irritating property, or a hot-melt adhesive melted by heat and applicable to the mesh-like sheet rather than the solvent-based adhesive in order to avoid even feeble skin irritating property.

In laminating the adhesive on the mesh-like sheet, examples of a method for maintaining air permeability can be a method of printing or transferring an adhesive to partially laminate an adhesive layer, and forming a non-laminated part as an air permeable part, a method of moving an adhesive in one direction or moving the adhesive suitably in two-dimensional directions such as moving the adhesive zigzag or forming the adhesive in dot shape, while describing a circle in filiform shape, so that gaps of the filiform adhesive maintain air permeability or moisture permeability or foam the adhesive, or a melt-blow method to form a layer.

Examples of an adhesive forming a non-hydrophilic adhesive layer can be an acrylic adhesive, a vinyl acetate adhesive (a vinyl acetate resin emulsion and an ethylene-vinyl acetate resin hot-melt adhesive), a polyvinyl alcohol adhesive, a polyvinyl acetal adhesive, a vinyl chloride adhesive, a polyamide adhesive, a polyethylene adhesive, a cellulose adhesive, a chloroprene (neoprene) adhesive, a nitrile rubber adhesive, a polysulfide adhesive, a butyl rubber adhesive, a silicone rubber adhesive, a styrene adhesive (for example, a styrene-based hot-melt adhesive), a rubber adhesive, a silicone adhesive, etc. Out of these adhesives, the rubber adhesive, the acrylic adhesive or the hot-melt adhesive is preferable for reasons of high adhesive strength, low cost, high long-term stability, and moreover a small fall in adhesive strength when heated, etc.

A tackifier such as petroleum resins represented by alicyclic petroleum resin such as rosins, cumarone-indene resin, hydrofined petroleum resin, maleic anhydride modified rosin, rosin derivatives or C5 petroleum resin, a phenolic tackifier (particularly a tackifier with an aniline point of 50°C or lower) such as terpene phenol resin, rosin phenol resin or alkyl phenol resin, a softener such as coconut oil, castor oil, olive oil, camellia oil or liquid paraffin, an antioxidant, a filler, aggregate, a tackiness modifier, a tackiness improver, a coloring agent, an antifoaming agent, a thickener, a modifier, etc. may be mixed in the adhesive by request.

Examples of the hot-melt adhesive can be known hot-melt adhesives with adhesiveness imparted, specifically a styrene adhesive containing an A-B-A type block copolymer such as SIS, SBS, SEBS or SIPS as a base polymer, a vinyl chloride adhesive containing vinyl chloride resin as a base polymer, a polyester adhesive containing polyester as a base polymer, a polyamide adhesive containing polyamide as a base polymer, an acrylic adhesive containing acryl resin as a base polymer, a polyolefin adhesive containing polyolefin such as polyethylene, ultra-low density polyethylene, polypropylene, ethylene-α-olefin and an ethylene-vinyl acetate copolymer, as a base polymer, a 1,2-polybutadiene adhesive containing 1,2-polybutadiene as a base polymer, or a polyurethane adhesive containing polyurethane as a base polymer, or adhesives formed of modified products thereof improved in adhesive property and changed in stability or the like, or a mixture of two or more of these adhesives. An adhesive layer composed of a foamed adhesive, or an adhesive layer composed of a cross-linked adhesive are also usable.

In the case of using the non-hydrophilic adhesive, a water-absorbing polymer may be mixed as a countermeasure to prevent a fall in adhesive strength caused by a moisture increase.

The hydrophilic adhesive is adequate if it contains a hydrophilic polymer or a water-soluble polymer as a main component and has adhesiveness and hydrophilic property.

The hydrophilic adhesive may contain the hydrophilic polymer such as polyacrylic acid, the water-soluble polymer such as sodium polyacrylate or polyvinyl pyrolidone, cross-linking agents such as dry aluminum hydroxide or metasilicate aluminate metal salt, and softeners such as glycerine or propylene glycol. The hydrophilic adhesive may also contain oil components such as high-grade hydrocarbon (for example, light liquid paraffin or polybutene), primary alcohol fatty acid ester (for example, isopropyl myristate), a silicon-containing compound (for example, silicone oil), fatty acid glycerine ester (for example, monoglyceride), or vegetable oil (for example, olive oil). The hydrophilic adhesive may further contain an antiseptic agent such as methyl parahydroxybenzoate or propyl parahydroxybenzoate, a solubilizer such as N-methyl-2-pyrolidone, a thickener such as carboxymethyl-cellulose, a surfactant such as polyoxyethylene cured castor oil or sorbitan fatty acid ester, a shaping agent such as oxycarboxylic acid (for example, tartaric acid), light anhydrous silicic acid, a water absorbing polymer or kaoline, a humectant such as D-sorbitol, a stabilizer such as edetate sodium, ester parahydroxybenzoate or tartaric acid, a cross-linking water-absorbing polymer, or a boron compound such as boric acid.

As a method of providing the mesh-like sheet with the heat generating member, the heat generating member and the mesh-like sheet can be integrally formed by partially using members that compose the heat generating member and the mesh-like sheet in combination, or adhesive layers may be provided on the rear face of the mesh-like sheet to stick the heat generating member thereto.

The adhesive layers 3a may be provided excluding rectangular regions 4b and 4c provided at a space in the longitudinal direction of the mesh-like sheet 3 as shown in Fig. 5.

In the case of the heat generating body 1 shown in Fig. 5, the adhesive layers 3a with predetermined width (for example, about 1 to 80 mm) are provided at predetermined spaces (for example, about 1 to 80 mm) including seal parts 2s1 and 2s1 at the peripheral edge of the wide surface of the heat generating member 2 at least in a region where the heat generating member 2 on the mesh-like sheet 3 are located, along the longitudinal direction of the heat generating body 1 (since the heat generating body shown in the figure has two heat generating members, the two heat generating members are regarded as one heat generating member in all). In this case, it is preferable to set a surface area ratio of the adhesive layer to 10 to 80% in a range excluding the seal parts 2s1 and 2s1 (including a seal part 2s2 other than the seal parts 2s1 at both ends) in the region where the heat generating member 2 is located, and to set a surface area ratio of the adhesive layer to 80% or more in a region where each of the seal parts 2s1 and 2s1 of the region is provided with the adhesive layer 3a. This is because an air permeability rate enough to obtain a sufficient sense of warmth as the heat generating body 1 can be obtained, and at the same time, the heat generating member 2 is firmly stuck to the mesh-like sheet 3 so as not to fall off. It is therefore further preferable to set the surface area ratio to 20 to 60% in the region excluding the seal parts and to set the surface area ratio to 90% or more in the region of each seal part.

It is preferable that regions 4b' and 4c' not provided with the adhesive layers 3a in the located region of the heat generating member 2 from the mesh-like sheet 3 are provided in strip shape as shown by dotted lines that indicate boundaries in Fig. 5. The whole heat generating body 1 can be warmed by narrowing the width of the strips, while air permeability at the substantially center part or the like of the heat generating member 2 is enhanced, and heat generation is ensured by making the width of at least part of the strip wide. The "strip shape" means to provide continuous striped shape from the mesh-like sheet 3 to the located region of the heat generating member 2. The width of the regions 4b and 4c where the adhesive layers 3a are not provided, is not particularly limited as long as the heat generating body 1 can be stuck to the skin without falling off during use and causing no severe pain or the like when stripping off the heat generating body 1. However, the width of the strip shape is preferably set to 1 to 60 mm, further preferably to 8 to 40 mm. The user thereby has no uncomfortable feeling during use, nor the user's skin is harmed when stripping off the heat generating body.

The regions 4b and 4c explained in Fig. 5 are formed in a direction orthogonal to the longitudinal direction of the heat generating body 1. Although it is not intended to exclude forming them in the longitudinal direction of the heat generating body 1, as shown in Fig. 5, the direction orthogonal to the expanding or extending direction of the mesh-like sheet 3 is preferable because, when the mesh-like sheet 3 extends, the regions 4b and 4c not provided with the adhesive layers 3a extend to relieve the uncomfortable feeling such as the stiff feeling caused by the movement of the body.

An explanation on the deformed example of Fig. 5 is made, referring to Fig. 6.

A heat generating body 1 in Fig. 6 is provided with three heat generating members continuously with seal parts 2s2 and 2s2 held between in the longitudinal direction of the heat generating body 1.

The adhesive layers are formed in a range of 10 to 80% of the surface area of the located region of the three heat generating members excluding the seal parts 2s1 and 2s1 on the mesh-like sheet 3. In this case, the adhesive layers are formed at the seal parts 2s2 and 2s2 extended in a direction orthogonal to the longitudinal direction of the heat generating body 1.

It is preferable to set the adhesive strength of the adhesive layers 3a to 2.0 to 3.3 N/10.5 mm and to set the shearing force to 6.0 to 8.5 kgf/29 mm. The adhesive strength is made low, and the shearing force is made high. The uncomfortable feeling such as the stiff feeling caused by the movement of the body is relieved, and the heat generating body can be prevented from falling off the body (the skin). It is more preferable to set the adhesive strength to 2.2 to 3.3 N/10.5 mm and to set the shearing force to 6.5 to 8.5 kgf/29 mm, and it is further preferable to set the adhesive strength to 2.2 to 2.8 N/10.5 mm and to set the shearing force to 6.5 to 8.0 kgf/29 mm. If the adhesive strength is less than 2.0 N/10.5 mm, although there is no problem if used as a sticking plaster, it is weak as adhesive strength for sticking the heat generating body with a certain degree of size and weight to the skin, which results in either failing to stick the heat generating body or making the heat generating body easy to fall off even if it is stuck. When the adhesive strength is 2.0 N/10.5 mm or more and the shearing force is less than 6.0 kgf/29 mm, the heat generating body is easy to fall off. When the adhesive strength exceeds 3.3 N/10.5 mm, particularly when the adhesive strength is 5.0 N/10.5 mm or more and the shearing force is 6.0 kgf/29 mm or more, the movement of the body causes the uncomfortable feeling such as the stiff feeling, and the heat generating body is not easily stripped off the skin when stripped off after use, to cause a pain when stripped off. Even if the sheering force exceeds 8.5 kgf/29 mm, if the adhesive strength is 3.3 N/10.5 mm or less, an uncomfortable feeling during use is small. However, the heat generating body is not easily stripped off the skin when stripped off after use, to cause a pain when stripped off.

A value obtained by sticking a test piece provided with an adhesive layer to an acrylic plate, and stripping the test piece off in a direction of 90° is used as the adhesive strength. A measuring method is as follows. The test piece with the adhesive layer formed in strip shape 10.5±1.0 mm wide and 100±5 mm long is prepared, and a release paper (a release film) on the adhesive surface of the test piece is peeled off to stick the test piece to the acrylic plate. A pressure bonding roller of 2 kg is rolled in one reciprocating motion thereon to bring the adhesive surface of the test piece and the acrylic plate into closer contact and to place them still for 30 minutes in a thermostatic chamber controlled to 20±3°C. Then the 90° strip adhesive strength is measured according to measuring conditions of JIS-Z0237. In measuring adhesive strength, a texture analyzer TA.XT2i (made by STABLE MICRO SYSTEMS LTD.) is used for a testing device, and the tensile moving speed in a length direction is set to 10±1 mm/min to obtain adhesive strength (N/10.5 mm). An acrylic plate (product name: COMOGLASS) (made by KURARAY CO., LTD.) is used as a test plate.

Shearing force is measured according to a testing method of JIS-K6850. A heat generating body 1 is left for 60 minutes in a thermostatic chamber (40±3°C) without being taken out of an outer bag. A stainless plate (2±1 mm thick, 29+1 mm wide and 110±3 mm long and its sample bond surface [29±1 mm wide x 33±3 mm long]) dried after washed with ethanol is also left in the same conditions. After the lapse of 60 minutes, the heat generating body 1 is taken out of the outer bag, and release paper (a release film) is peeled off. An adhesive layer 3a part is adjusted to the sample bond surface, and a pressure bonding roller of 2 kg is rolled in one reciprocating motion to prepare a test piece. In measuring shearing force, a tension testing machine with a thermostatic bath (made by TESTER SANGYO CO., LTD.) is used for a testing device, and measurement is made at a tensile moving speed of 300±30 mm/min in the length direction. A maximum load when the adhesive layer 3a is stripped from the stainless plate is measured to obtain shearing force (kgf/29 mm). The stainless plate (SUS304 specified in JIS-G4305) is used as a testing plate

An explanation on members that compose the heat generating body is made. It is preferable to cover the contact surface of the heat generating member 2 in Fig. 1 and Fig. 2 with a protective sheet.

The protective sheet is provided for preventing the direct contact of the heat generating member with the skin and is not particularly limited on material or the like as long as it is sufficient for attaining this purpose. One example may be fibers such as absorbent cotton, nonwoven fabric, woven fabric, paper or pulp. A laminate of one or more kinds of them, a laminate of a polyethylene film etc. thereon, or the fibers etc., a laminate of the fibers or a laminate of the polyethylene film etc. being formed into bag shape, or the bag shape filled with fibers etc. of sheet shape, crushed shape, etc., may be suitably used.

When the surface side, contacting the skin, of the heat generating member 2 is placed in a wet state, a feeling of use may be impaired, or heat generating characteristics may change. In order to avoid this, it is preferable to select material with moisture absorbing property such as rayon for the protective sheet out of the enumerated materials

The protective sheet may be formed by laminating a plurality of sheets according to a user. In this case, the protective sheet may be provided as an addition to a protective sheet already provided at the heat generating member. The user can thereby adjust the temperature to a suitable temperature to further enhance low temperature burn preventing property.

It is preferable to provide the exposed surface of the protective sheet with protrusions. An air layer is formed between the contact surface side of the heat generating member and the protective sheet to cause a heat insulating effect, and the sense of warmth can be adjusted even with the comparatively thin protective sheet. The protrusions can be provided in a manufacturing process, and cost can be low. In the case of providing the protective sheet with the protrusions, the size (the maximum outer diameter of the protrusion in a plan view can be made about 1 to 30 mm, for example), number, etc. of the protrusions are not particularly limited as long as the user's sense of warmth can be adjusted. The shape of the protrusions such as plane shape is not particularly limited, and may be a round shape such as a circular or elliptical shape, or a polygonal shape such as a triangular or quadrilateral shape. The height of the protrusions is not particularly limited unless they give an uncomfortable feeling in using the heat generating body, and may be about 0.3 to 2.0 mm, for example. If it is less than 0.3 mm, the heat insulating effect is hardly produced, and there is no change in the effect of adjusting the sense of warmth compared with the protective sheet not provided with the protrusions. In the case of exceeding 2.0 mm, there is a risk of breaking the protective sheet stretched out in pressure forming. If the height of the protrusions is preferably set to 0.3 to 1.5 mm, more preferably to 0.3 to 1.0 mm, the excellent effect of adjusting the sense of warmth can be obtained, and the protective sheet is never damaged. The line speed of a production process can thereby be kept comparatively high to attain efficient production.

The heat generating member 2 is not particularly limited as long as it is a member that generates heat to the extent of not causing a burn, and can be formed, for example, by holding a heat generating composition between a covering material located on the mesh-like sheet 3 side, and a base material located on the side opposite to the mesh-like sheet 3, and enclosing the heat generating composition between the covering material and the base material so as to form a seal part with a predetermined width (for example, about 3 to 15 mm) at the outer peripheral parts of the covering material and base material.

An air permeable material is usable for the base material, but it is preferable to form the base material of an air impermeable material. This is because the air impermeable material has sufficient strength without providing holes or the like for obtaining air permeability, and a damp feeling caused by restraining the release of water vapor is not given. The air impermeable material is to have an air permeability rate of 0 ml/min/506.7 mm² or less in this description.

The base material is not particularly limited as long as it has sufficient strength not to be torn or the like during use and is stickable to the covering material with sufficient sealing strength using a heat seal or the like and further does not have a texture to cause an unpleasant feeling in the case of contacting the user's skin.

A resin film may be provided as the base material on the heat generating member side of the protective sheet. In the case of forming the protective sheet of a nonwoven fabric, for example, if material with a resin film such as a polyethylene film laminated as the base material on the nonwoven fabric is used from the time of manufacturing the heat generating member, a process for providing the heat generating member with the protective sheet can be reduced.

The covering material is not particularly limited as long as it has air permeability. A laminate of an air permeable resin film (a porous film or the like, or an air permeability controllable film or the like) may be used as the covering material used in the present invention, and even a laminate of the nonwoven fabric and porous film may be used.

Although there is no particular limitation also on a method for manufacturing the heat generating member, the covering material and the base material are formed in bag shape by heat sealing or the like, and a heat generating composition measured to a predetermined weight is put therein. All sides of the bag are then sealed by sealing or the like.

The sealing can be carried out by heat sealing with a heat roll, pressing from the upper and lower sides, etc. or applying an adhesive such as a hot-melt with a brush, a roll, or the like or spraying the adhesive, or the like.

A heat generating composition containing a heat generating substance such as iron, a carbon component, a reaction accelerator and water as essential components and causing a heat generating reaction in contact with air can be used as the heat generating composition.

The heat generating composition may contain at least one kind selected from additional components including water holding agent, a water absorbing polymer, a pH adjustor, a hydrogen generation inhibitor, aggregate, fibrous materials, a functional substance, a surfactant, an organic silicon compound, a pyroelectric substance, a humectant, a fertilizer component, a hydrophobic polymer compound, heat generating accelerator, metals other than iron, metallic oxide other than iron oxide, acidic substances, or a mixture thereof.

It is preferable to provide the adhesive layer with a release sheet in order to prevent unintended sticking of the adhesive layer. Release paper or film used for an ordinary sticking tape or the like can be used as the release sheet.

An example of providing regions 6a and 6b with no adhesive layer 3a across a located region of the mesh-like sheet 3 and heat generating member 2 of the heat generating body 1 and providing the release sheets 7 excluding the regions 6a and 6b is shown in Fig. 7.

A heat generating body 1 shown in Fig. 8 indicates a deformed example of a method of providing the release sheets 7. Regions 6a and 6b with no adhesive layer 3a are provided in regions of heat generating members located on both sides of three heat generating members arranged in the longitudinal direction of the heat generating body 1, and the center side ends of release sheets 7a and 7b located on both sides are folded to provide folded parts 8a and 8b.

The release sheet may be provided on the whole rear face of the heat generating body, and further two or more release sheets may be provided to partially overlap on the centerline in the longitudinal direction of the heat generating body or in a direction orthogonal to the longitudinal direction or in a position where the heat generating member is present. One release sheet or both release sheets are provided with folds, and a predetermined clearance (for example, about 1 to 40 mm) is provided between the respective release sheets according to the width of a strip not provided with the adhesive layer of the mesh-like sheet between the two or more release sheets. Consequently, when the user tries to stick the heat generating body to the skin, the release sheets are easily peeled to facilitate sticking to the skin so as to be more convenient. In the case of providing the clearance between the release sheets, since no adhesive layer is provided between the release sheet and the heat generating member, the user can peel the release sheet by putting the finger between the release sheet and the heat generating member. The release sheet is thereby peeled extremely easily, which is desirable.

It is preferable to contain a medicine on the outside of the heat generating body, and further it is preferable to provide a medicine layer in a position adjacent to the heat generating body.

Specifically, examples of a method of containing the medicine may be applying the medicine by spray, arranging (for example, laminating) a sheet or the like containing the medicine in absorbent cotton, a nonwoven fabric, or the like, adjacently to the heat generating body.

The medicine is not limited as long as it is a substance with functions, and examples thereof can be at least one kind selected from aromatic compounds, vegetable extracts, crude drugs, perfumes, slimming agents, analgesics, blood circulation promoters, swelling improvers, antibacterial agents, sterilizers, mold inhibitors, odor eaters, deodorants, percutaneously absorptive drugs, fat-splitting components, minus ion generators, far infrared ray radiants, magnetic bodies, fomentations, cosmetics, bamboo vinegar, wood vinegar, and the like.

Specific examples thereof include aromatic compounds (for example, menthol and benzaldehyde), vegetable extracts (for example, mugwort extract), crude drugs (for example, moxa), perfumes (for example, lavender and rosemary), slimming agents (for example, aminophylline and tea extract), analgesics (for example, indomethacin and *dl*-camphor), blood circulation promoters (for example, acidic mucopolysaccharide and chamomile), swelling improvers (for example, horse chestnut extract and flavone derivatives), fomentations (for example, aqueous boric acid, physiological saline, and aqueous alcohols), fat-splitting components (for example, jujube extract, caffeine, and tonalin), cosmetics (for example, aloe extracts, vitamin preparations, hormone preparations, anti-histamines, and amino acids), antibacterial agents and sterilizers (for example, carbolic acid derivatives, boric acid, iodine preparations, invert soaps, salicylic acid based substances, sulfur, and antibiotics), and mold inhibitors.

The percutaneously absorptive drug is not particularly limited as long as it has percutaneous absorption. Examples thereof include corticosteroids, anti-inflammatory drugs, hypertension drugs, anesthetics, hypnotic sedatives, tranquillizers, antibacterial substances, antifungal substances, skin stimulants, inflammation inhibitors, anti-epileptics, analgesics, antipyretics, anesthetics, sterilizers, antimicrobial antibiotics, vitamins, antiviral agents, swelling improvers, diuretics, antihypertensives, coronary vasodilators, anti-tussive expectorants, slimming agents, anti-histamines, antiarrhythmic agents, cardiotonics, adrenocortical hormones, blood circulation promoters, local anesthetics, fat-splitting components, and mixtures thereof. However, it is not limited to these examples. These drugs are used singly or in admixture of two or more kinds thereof if necessary.

The content of the medicine is not particularly limited as long as it is in a range where the medicinal effect can be expected. However, from the viewpoint of medicinal effect, economical efficiency, etc., the content of the medicine is preferably 1 to 1,000 mg., further preferably 10 to 500 mg. for one heat generating body.

The medicine may contain a perfume as mentioned above.

Examples of the perfume can be a natural perfume and a synthetic perfume. Examples of the natural perfume can be, for example, an animal perfume (for example, musk, civet, ambergris, or the like) and a vegetable perfume (for example, almond oil, cinnamon oil, citronella oil, cognac oil, oil garlic, ginger oil, grapefruit oil, hop oil, lemon oil, nutmeg oil, mustard oil, peppermint oil, orange oil, or the like), and examples of the synthetic perfume can be, for example, hydrocarbons (limonene or the like), alcohols (citronellol or the like), phenols (eugenol or the like), aldehydes (cinnamic aldehyde or the like), ketones (camphor, P-methylacetophenone, or the like), a lactone (coumarin or the like), or esters (ethyl myristate, cinnamyl cinnamate, methyl anthranilate, or the like), but the examples are not limited to these perfumes.

The medicine may be the above-mentioned essential oil components.

Except the above-mentioned components, the medicine may contain aldehydes such as hexyl cinnamic aldehyde, 2-methyl-3-(4-tert-butylphenyl)-propanal, 4-(4-hydroxy-4-methylpentyl)-3-cyclohexene-1-carboxyaldehyde, and vanillin; phenols such as anethole and eugenol; and lactones such as γ-nonalactone and γ-undecalactone.

It is preferable that at least 50 mass% of one or more kinds of the above exemplified active components is mixed in the perfume composition.

A solvent or the like may be mixed in the perfume composition. As a usable solvent, dipropylene glycol, ethyldiglycol, isopropyl myristate, benzyl benzoate, triethyl citrate, diethyl phthalate, and the like can be used.

The applied amount of the perfume composition, although depending on the kind, is preferably 50 to 500 mg., further preferably 100 to 300 mg. for one heat generating body as a general range to obtain a fully satisfactory result.

Material that can retain a liquid perfume composition is used as a formed sheet material. A fiber material such as paper, absorbent cotton, nonwoven fabric, or woven fabric is suitably used as such a material. The sheet material may be of the same shape as the heat generating body or may be larger or smaller than the outside dimensions of the heat generating body.

The state of the perfume in a liquid substance may be a dissolved or dispersed state etc. and is not particularly limited.

The above-mentioned medicine and perfume may be placed on either the front side or the rear side of the heat generating body.

The "nonwoven fabric" in the present invention means a sheet with air permeability, extensibility or expansibility and base material strength and is not particularly limited on material or the like. Specific examples usable as the "nonwoven fabric" can be an air-permeable laminate of independent or two or more kinds of nonwoven fabric, an air-permeable laminate of at least one kind of an air permeable film such as a porous film or a perforated film, and paper or the like, an air permeable material obtained by using a needle or the like to provide pores in an air impermeable wrapping material with a polyethylene film laminated on a nonwoven fabric, a nonwoven fabric with fibers laminated, thermocompressed and controlled in air permeability, etc. The perforated film is an air permeable film obtained by providing pores in an air impermeable film such as a polyethylene film using a needle. The nonwoven fabric provided at the outer periphery of the heat generating member is provided with the adhesive layer for use. The heat generating body can thereby be inexpensively stuck to the skin.

Examples of material of the nonwoven fabric include vegetable fibers such as pulp, hemp, cotton, rayon, and acetates; synthetic pulps made of polyethylene, etc. as the raw material; single fibers or composite fibers of thermoplastic polymer substances including polyolefin bases aiming a self welding type, such as polyethylene, polypropylene, copolymers composed mainly of propylene and ethylene, and propylene-ethylene-butene ternary random copolymers, polyamide bases such as nylon 6, and polyester bases such as polyethylene terephthalate; mixed fibers thereof; and mixtures thereof with cellulose fibrous pulp, etc. With respect to the nonwoven fabric, although short fiber nonwoven fabrics, long fiber nonwoven fabrics, and continuous filament nonwoven fabrics can be used, long fiber non-woven fabrics and continuous filament nonwoven fabrics are preferable in view of mechanical properties. From the standpoint of production process, a dry nonwoven fabric, a wet nonwoven fabric, a spun bond, a spunlace, a thermal bond, and the like can be used.

### Examples

As the heat generating composition that composes the heat generating member used in the following Examples 1 to 6 and 8 to 10, 12.5 g of a composition was used, the composition containing, per 100 pts.wt. iron powder, 25.2 pts.wt. active carbon, 3.3 pts.wt. water absorbing polymer, 0.6 pts.wt. pentasodium triphosphate which is a pH adjustor, and 60.0 pts.wt. of an 8 wt.% aqueous solution of sodium chloride which is a reaction accelerator.

The heat generating composition was formed to be 60 mm long, 65 mm wide and 2.5 mm thick for use. In Example 4, the heat generating composition with a weight of about 7g formed to be 50 mm long, 50 mm wide and 2.5 mm thick was used.

### Example 1

The heat generating composition was placed on a base material formed by laminating polyethylene 70 mm long, 75 mm wide and 40 µm thick on a nylon nonwoven fabric (weight per unit area of 40 g/m²) 70 mm long and 75 mm wide, and the top of the heat generating composition was covered with a covering material formed by laminating a porous film of polyethylene 100 mm long, 115 mm wide and 70 µm thick on a mesh-like nonwoven fabric (weight per unit area of 40 g/m²) 100 mm long and 115 mm wide. The base material and the covering material were sealed at the peripheral edge of the heat generating composition to form a heat generating member. An adhesive composed of an acrylic solvent-based adhesive was applied (an applied thickness of 23 µm) onto the covering material outside of the seal to form a heat generating body in Example 1.

### Example 2

A protective sheet 80 mm long, 85 mm wide and 0.38 mm thick was provided on the heat generating member of the heat generating body in Example 1 to form a heat generating body in Example 2.

The protective sheet in use is structured to laminate a polyethylene film with a thickness of 28 µm on a metallocene polyethylene film with a thickness of 12 µm and to apply an acrylic adhesive thereon with an applied amount of 6±1 g/m² to laminate a nonwoven fabric with 100% rayon with a weight per unit area of 55±5 g/m², and the protective sheet was provided on the heat generating member so that the nonwoven fabric was located on the sensor side of a tester.

Temperature was measured under the following measuring conditions.

### [Temperature measuring test of the heat generating part]

### (A) Testing condition

- Environmental temperature: 20±1.5°C
- Placing table: a heater having a hose connecting part for circulating warm water with a height of 100 mm (a volume of 16.42 L) was placed on styrene foam 300 mm long, 600 mm wide and 30 mm thick (from a floor face), and the top face material of the heater was SUS304 of JIS-G4340 formed into a plate-like member with a thickness of 3 mm. Further, 5.0 g (27.8 g/m²) of white vaseline was applied to the surface of the upper face of the plate-like member, and a urethane rubber sheet with a thickness of 5 mm and a hardness of 60° was placed thereon so that the least possible air entered. A circulating thermobath was used for circulating warm water, and warm water was circulated through the heater at the flow rate of 8±2 L/min to regulate the surface of the urethane rubber sheet to 33±1.0°C.
- Temperature sensor: made by Anritsu Meter Co., Ltd., tape-type, specification temperature range: -50 to 210°C, accuracy: ±0.5°C
- Data logger: made by ADVANTEST CORPORATION, TR2114H
- Measuring interval: 5 minutes

### (B) Testing method

A temperature sensor was stuck onto the heat generating member or onto the protective sheet provided on the heat generating member of the heat generating body in Examples 1 to 6 and 10 left in the above-mentioned environmental temperature for 1 hour or longer, and the heat generating body was stuck onto the placing table, placing the stuck side of the temperature sensor at the bottom side. Two sheets of a flannel covering material (flannel with a thickness of 0.70±0.15 mm, 100% cotton, a warp of No.20 count/single yarn, and a weft of No.20 count/2 ply yarn) were put thereon, and recording of the data logger was started after three minutes from the start of heat generation to measure the temperature.

Graphs indicating the measured results of the above Examples 1 and 2 are shown in Fig. 9.

In Examples 1 and 2, it is proved that the heat generating member does not directly come in close contact with the skin through the adhesive, and the burden on the skin is eased even under continuous use to cause no low temperature burn. It is also proved that the heat generating body in Example 2 provided with the protective sheet relieves even more the burden on the skin.

As the actual feeling of use, no uncomfortable feeling was given in either of Examples 1 and 2.

### Example 3

An explanation is made on an example of changing the weight per unit area and material of the nonwoven fabric to inspect the effectiveness of the protective sheet.

A heat generating composition was placed on a base material formed by laminating a polyethylene resin film with a thickness of 40 µm on a rayon spunlace nonwoven fabric (weight per unit area of 45 g/m²) 70 mm long and 75 mm wide, and the top of the heat generating composition was covered with a covering material formed by laminating a porous film made of polyethylene resin with a thickness of 70 µm, on a PET spunlace nonwoven fabric (weight per unit area of 30 g/m²) 70 mm long and 75 mm wide. The base material and the covering material were then sealed at the peripheral edge of the heat generating composition to form a heat generating member. An adhesive (23 µm in applied thickness) formed of an acrylic solvent-based adhesive was applied to a mesh-like sheet (weight per unit area of 40 g/m²) made of a PET spunlace nonwoven fabric 100 mm long and 125 mm wide, in the width of 10 mm at 10 mm spaces in a TD direction, and the covering material side of the heat generating member was stuck to the applied surface of the adhesive. At this time, the respective longitudinal and lateral centerlines of the heat generating member and the mesh-like sheet provided with an adhesive layer were superimposed, and the following materials a to c 80 mm long and 85 mm wide were provided on the contact surface (base material) side of the heat generating members respectively as protective sheets to form heat generating bodies.
a) PET spunlace nonwoven fabric (weight per unit area of 40 g/m²) made by SHINWA Corp.
b) PET spunlace nonwoven fabric (weight per unit area of 60 g/m²) made by SHINWA Corp.
c) Rayon spunlace nonwoven fabric (weight per unit area of 40 g/m²) made by SHINWA Corp.

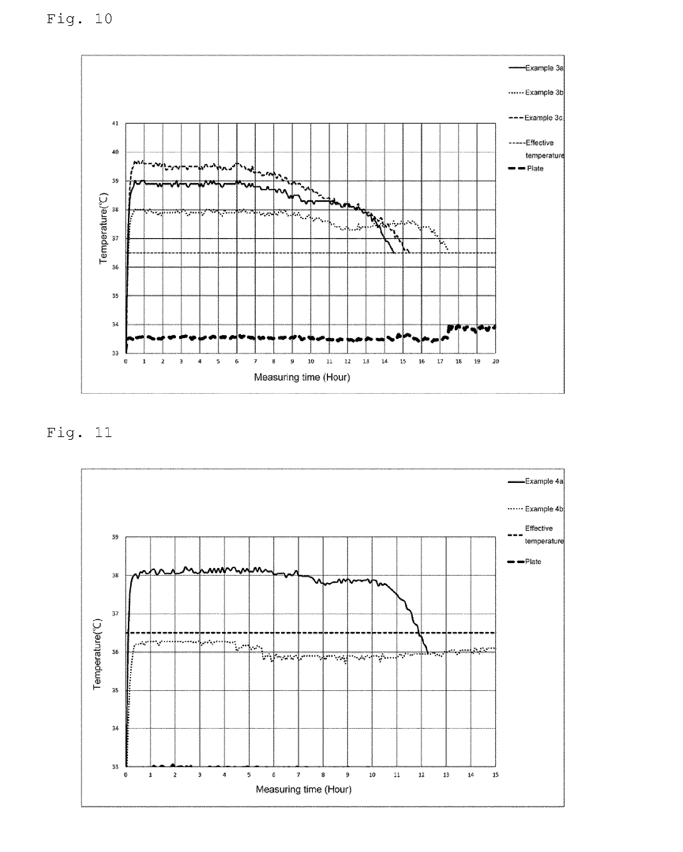

The respective heat generating bodies are made Examples 3a to 3c, and the temperature changes thereof are shown in Fig. 10. The "effective temperature" in the graphs is an example of a standard temperature as the heat generating body, and the "plate" shows the temperature change of a sensor with no temperature measuring object (the heat generating body) placed thereon on the placing table (the surface of the urethane rubber sheet) to which the sensor when measuring is mounted, and are used in the same meaning on the graphs shown blow.

It is proved from the graph that the heat generating body effectively functions for 14 hours whichever material of nonwoven fabric is used, and the temperature can be regulated by the material and the difference of the weight per unit areas according to the user's sense of warmth. The protective sheets are laminated for use so as to be adjustable to each user's suitable sense of warmth.

### Example 4

An explanation is made on the examples of a heat generating body in which the covering material side of the heat generating member was stuck to the mesh-like sheet, and a heat generating body in which the base material side of the heat generating member was stuck to the mesh-like sheet.

A heat generating composition was placed on a base material formed by laminating a polyethylene resin film with a thickness of 40 µm on a rayon spunlace nonwoven fabric (weight per unit area of 45 g/m²) 60 mm long and 60 mm wide, and the top of the heat generating composition was covered with a covering material formed by laminating a porous film (70 µm in thickness) made of polyethylene, on a PET spunlace nonwoven fabric (weight per unit area of 30 g/m²) 60 mm long and 60 mm wide. The base material and the covering material were then sealed at the peripheral edge of the heat generating composition to form a heat generating member. An adhesive (23 µm in applied thickness) formed of an acrylic solvent-based adhesive was applied to a mesh-like sheet (weight per unit area of 40 g/m²) made of a PET spunlace nonwoven fabric 90 mm long and 100 mm wide, in the width of 10 mm at 10 mm spaces in a TD direction, and the applied surface of the adhesive was provided with a release sheet.

The release sheet provided at the mesh-like sheet was removed, and the covering material side of the heat generating member was stuck to the applied surface of the adhesive to form a heat generating body which was made Example 4a. In the same way, the base material side of the heat generating member was stuck to the applied surface of the adhesive to form a heat generating body which was made Example 4b. At this time, the respective longitudinal and lateral centerlines of the heat generating member and the mesh-like sheet provided with an adhesive layer were superimposed.

The average temperature change of sticking surfaces (plate surfaces) of the heat generating bodies in Examples 4a and 4b are shown in Fig. 11. It is proved from the graph that Example 4b indicates a slightly lower value than the effective temperature, but heat generation can be stably obtained for about 11 hours in either example. It is proved from the graph of Example 4a that the heat generating effect of the heat generating body in which the covering material side of the heat generating member is stuck to the mesh-like sheet is high. According to the use part and the sense of warmth of the user, the sticking surface to the mesh-like sheet may be changed to the covering material side or the base material side even in the same heat generating member, and the sense of warmth of the heat generating body can be changed.

### Example 5

The mesh-like sheet of the heat generating body of Example 3 (wherein a protective sheet was not provided) was formed of the following materials (the dimensions were the same as those of Example 3) to prepare heat generating bodies respectively, and temperature characteristics were measured.
a) PET spunlace nonwoven fabric (weight per unit area of 40 g/m²) made by SHINWA Corp.
b) PET spunlace nonwoven fabric (weight per unit area of 45 g/m²) made by Yuho Co., Ltd.
c) Composite spunlace nonwoven fabric (weight per unit area of 40 g/m²) containing 60% of Rayon, 20% of PET and 20% of binder fiber made by Daiwabo Polytec Co., Ltd.

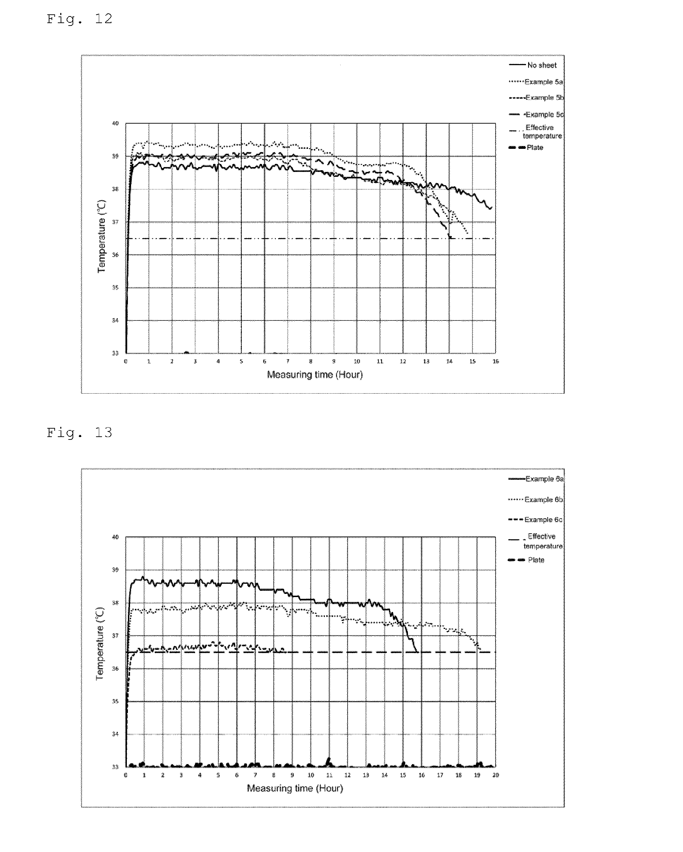

The respective heat generating bodies were made Examples 5a to 5c, and the temperature changes are shown in Fig. 12.

It is proved from Fig. 12 that a heat generating effect for at least 14 hours can be obtained whichever material of a) to c) is used to form the mesh-like sheet.

### Example 6

Heat generating bodies were respectively formed of the following materials (dimensions were the same as those in Example 3) instead of a protective sheet of the heat generating body in Example 3, and temperature characteristics were measured.
a) No protective sheet
b) Two protective sheets (each structured to laminate a polyethylene film with a thickness of 28 µm on a metallocene polyethylene film with a thickness of 12 µm and to apply an acrylic adhesive thereon with an applied amount of 6±1 g/m² to laminate a nonwoven fabric of 100% rayon with a weight per unit area of 55±5 g/m²) in Example 2 were prepared. The metallocene polyethylene surfaces were placed to face each other, and three sides of the peripheral edges were heat-sealed to form bag shape. Then 0.9 g of flocculent pulp (bulk density of 0.1 g/cm³) was put in the bag, and the remaining side was heat-sealed to form a protective sheet of flat bag shape 80 mm long, 85 mm wide and 2.5 mm thick.
c) In the same way as the above b, 2.2 g of flocculent pulp was put in a bag to form a protective sheet of flat bag shape of 80 mm long, 85 mm wide and 5.0 mm thick.

The respective heat generating bodies were made Examples 6a to 6c, and the temperature changes are shown in Fig. 13.

It is proved from the graph that a heat generating effect can be obtained for 14 hours at a minimum including Example 6c in which the effect lasts for many hours within a range of 36 to 37°C whichever material of a) to c) the protective sheet is formed of.

### Example 7

The following materials (dimensions were 100 mm long and 100 mm wide) were used to make measurement on an air permeability rate (measured in Φ75 mm) in the case of superimposing the nonwoven fabric side of a covering material (the following material d)) on a mesh-like sheet (the following materials a) to c) without providing an adhesive layer) and in the case of only the covering material.
a) PET spunlace nonwoven fabric (weight per unit area of 40 g/m²) made by SHINWA Corp.
b) PET spunlace nonwoven fabric (weight per unit area of 45 g/m²) made by Yuho Co., Ltd.
c) Composite spunlace nonwoven fabric (weight per unit area of 40 g/m²) containing 60% of Rayon, 20% of PET and 20% of binder fiber made by Daiwabo Polytec Co., Ltd.
d) A covering material formed by laminating a porous film made of polyethylene resin with a thickness of 70 µm on PET spunlace nonwoven fabric (weight per unit area of 30 g/m²) made by NITTO LIFETEC CORPORATION

The measured results on the materials a) to d) without providing an adhesive layer were 7.4, 7.3, 7.4, 7.5 (ml/min/506.7 mm²) respectively, and the covering material superimposed on the mesh-like sheet made no great difference in the air permeability rate from only the covering material.

Regarding the materials a) and d), the measured result on the air permeability rate in the case of applying an acrylic solvent-based adhesive in a thickness of 23 µm and a width of 10 mm at spaces of 10 mm on one-side surface of a) to stick a nonwoven fabric surface of d) was 7.3 (ml/min/506.7 mm²), which proves that there is not much change in the air permeability rate even if providing the adhesive at a surface area ratio of 50% between the mesh-like sheet and the covering material, thereby having no influence on heat generating characteristics.

### Example 8

The adhesive layer was formed at the rate of 50% on one-side surface of the mesh-like sheet of the heat generating body in Example 5a to form a heat generating body of Example 8, and shearing force (kgf/29 mm) and adhesive strength (N/10.5 mm) were measured.

The same measurement was made on a product with an adhesive layer on the market for comparison.

The results are shown in the following table.

A feeling of use of the product of Example 8 was excellent in follow-up performance to an attached surface with no stiff feeling.

**Table 1**

| Manufacturer, product name, etc. | Shearing force | Adhesive strength |
|---|---|---|
| Example 8 | 7.06 kgf | 2.53 N |
| ThermaCare for shoulders made by WYETH (company) | 6.48 kgf | 9N |
| HeatWraps made by MEDIHEAT (company) | 2.57 kgf | 8.0 N |
| TYLENOL PRECISE | 4.03 kgf | 3.5 N |
| Adhesive bandage called DERUGADO (product number: DD-51) made by ASO Pharmaceutical Co., Ltd. | 4.60 kgf | 1.512 N |
| MEDICARE KABUSETE GADO I made by Morishita Jintan Co., Ltd. | 4.05 kgf | 1.598 N |
| Nurseban Touch made by KOYO SANGYO Co., Ltd. | 1.75 kgf | 0.980 N |
| Surgical Tape YOKUNOBI (product number: NHB38) made by Nichiban Co., Ltd. | 2.85 kgf | 2.554 N |
| NIKKOBAN (No. 127) made by NIKKO YAKUHIN KABUSHIKI KAISHA | 9.85 kgf | 3.445 N |

### Example 9

a) The heat generating body of Example 3a was made a heat generating body of Example 9a.
b) A covering material (formed by laminating a porous film made of polyethylene resin with a thickness of 70 µm on a PET spunlace nonwoven fabric (weight per unit area of 30 g/m²) 70 mm long and 75 mm wide) was used instead of a base material (formed by laminating a polyethylene resin film with a thickness of 40 µm on a rayon spunlace nonwoven fabric (weight per unit area of 45 g/m²) 70 mm long and 75 mm wide) of the heat generating body of Example 9a, to form a heat generating body with double side air permeability. Except this, the same configuration as Example 9a was applied to form a heat generating body of Example 9b.
c) Instead of the protective sheet (the PET spunlace nonwoven fabric (weight per unit area of 40 g/m²) made by SHINWA Corp.) of the heat generating body of Example 9a, a sheet used as the protective sheet in Example 2 (structured to laminate a polyethylene film with a thickness of 28 µm on a metallocene polyethylene film with a thickness of 12 µm and to apply an acrylic adhesive thereon with an applied amount of 6±1 g/m² to laminate a nonwoven fabric with 100% rayon with a weight per unit area of 55±5 g/m²) was provided such that the polyethylene surface of the protective sheet was provided on the contact surface (base material) side of the heat generating member and that the rayon nonwoven fabric contacted the skin side of the body. Except this, the same configuration as Example 9a was applied to form a heat generating body of Example 9c.

Temperature measurement on the body (the abdomen and the skin surface) of an examinee (user) of Example 9 was made under the following measuring conditions.

### [Temperature measurement of the heat generating body on the body]

### (A) Testing conditions

- Environmental temperature: 25±3°C (a temporary shift was made to an environmental temperature outside a range for a short time)
   (After 7 to 8 hours from the start of use, a shift was made to an environmental temperature: 20±3°C for several minutes.)
- A temperature sensor: made by Anritsu Meter Co., Ltd., SE0023, tape-type, specification temperature range: -50 to 250°C, accuracy: ±2.5°C
- A data logger: made by Anritsu Meter Co., Ltd., AM-8010E (Type E)
- Measuring intervals: one minute

### (B) Measuring method

The heat generating body 1 used in the present invention was used on the body (the abdomen with the skin temperature of 35±2°C) of the examinee to make temperature measurement. The heat generating body 1 sealed in a bag was taken out, and a release sheet was quickly peeled off. The temperature sensor was stuck onto the protective sheet provided on the heat generating member of the heat generating body. The stuck side of the temperature sensor was stuck to the skin surface of the body (the abdomen) of the examinee, and when the temperature sensor was stuck, recording of the data logger was started to measure the temperature.

When measuring, "a shirt of 100% cotton" and "a polo shirt of 95% PET and 5% cotton" were worn on the skin to which the heat generating body 1 was stuck, from the skin side for measurement.

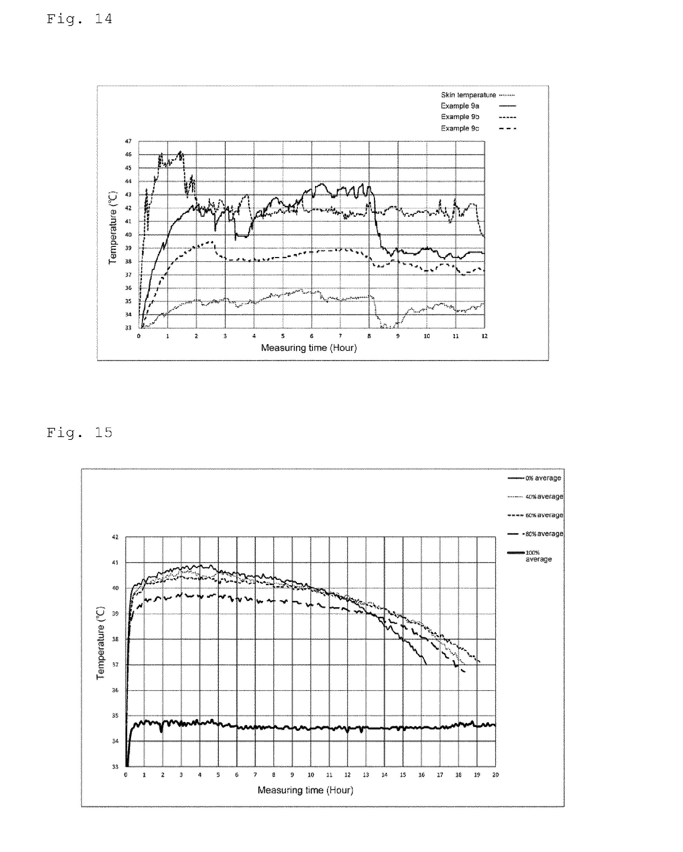

The measured results of the temperatures on the sticking surface side to the skin of the above Examples 9a to 9c are shown in Fig. 14.

It is proved from the graph of Fig. 14 that Example 9a is desirable from the viewpoint of a heat generating time and temperature.

However, although 8 hours or more have passed after the start of use, when the user moved to a place where the environmental temperature was low, the measured temperature suddenly dropped. On the other hand, the heat generating body of Example 9b kept a comparatively high temperature for 1 to 2 hours after the start of use, and it may be too hot for the user depending on the applied part and the sense of warmth of the user. However, this is adjustable by suitably using the protective sheet, and there was no temperature drop like Example 9a by forming the heat generating member to be air permeable on both sides. The heat generating body of Example 9c showed a lower temperature than Example 9a through the applied part and proved to be effective in temperature regulation as the protective sheet.

### Example 10

In order to examine the relationship between the area of the adhesive layer provided at the mesh-like sheet and the heat generating characteristics of the heat generating body, the measured result of heat generating characteristics by changing the adhesive layer within a range of 10 to 100% in the positions of the following a) to c) instead of the adhesive layer applied to the mesh-like sheet of the heat generating body of Example 5a is shown in a graph. Except this, the same configuration as Example 5a was applied to form a heat generating body of Example 10.
a) The mesh-like sheet was provided with adhesive layers in strip shape in the same width at both ends (including seal parts) and the center part in the longitudinal direction of the heat generating member.
b) The mesh-like sheet was provided with adhesive layers in strip shape in the same width at both ends (including the seal parts) in the longitudinal direction of the heat generating member.
c) The mesh-like sheet was provided with an adhesive layer in strip shape only at the center part in the longitudinal direction of the heat generating member.

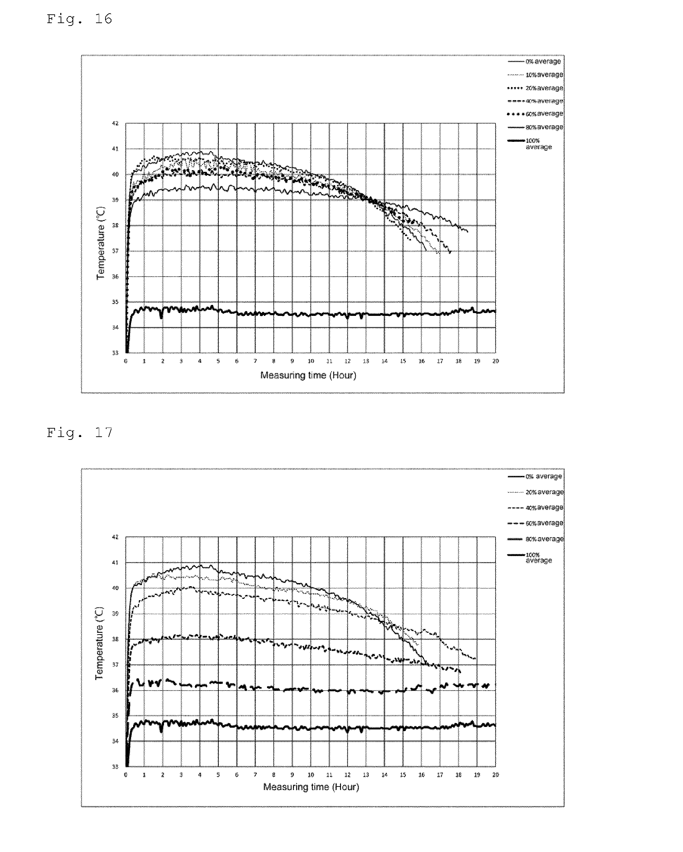

The respective cases were made Examples 10a to 10c and are shown in graphs in Fig. 15 to Fig. 17.

It is proved that there is no particular impediment to heat generating characteristics even if the adhesive layers are provided as in Examples 10a and 10b excluding the case of providing the adhesive layer at 100% to cover the covering material side surface of the heat generating member or the case of providing the adhesive layer at the rate of 60% or more only at the center part as in Example 10c.

Even in the case of providing the mesh-like sheet with the adhesive layer at 100%, air permeability can be obtained by perforation because the same tendency was obtained as a result of measuring temperature characteristics.

### Description of Reference Numeral

- 1: Heat generating body
- 2: Heat generating member
- 2s: Seal part
- 3: Mesh-like sheet
- 3a: Adhesive layer
- 4b and 4c: Region not provided with adhesive layer
- 5: Hole (opening)
- 6a and 6b: Region not provided with adhesive layer
- 7 (7a and 7b): Release sheet
- 8a and 8b: Folded part of release sheet

## Claims

1. A heat generating body **characterized in that** a heat generating member having a wide surface is provided on a mesh-like sheet larger in outer shape than the one-side wide surface of the heat generating member, and an adhesive layer is provided on the mesh-like sheet located at the outer peripheral part of the heat generating member.

2. The heat generating body according to claim 1, wherein the mesh-like sheet is provided with holes.

3. The heat generating body according to claim 1, wherein the adhesive layer is formed on the whole one-side surface of the mesh-like sheet, and the heat generating member is stuck to the mesh-like sheet through the adhesive layer.

4. The heat generating body according to claim 1, wherein the adhesive layer is formed in a range of 10 to 80% of the surface area of a region, in which the heat generating member is located, on the mesh-like sheet (excluding a seal part when the seal part is formed at the peripheral edge of the wide surface of the heat generating member), and the adhesive layer is formed at the seal part of the heat generating member extending in an orthogonal direction to the longitudinal direction of the heat generating body.

5. The heat generating body according to claim 4, wherein a region surrounded by the heat generating members located on the outermost peripheral edge is made the region (excluding the seal parts of the heat generating members located at both ends in the longitudinal direction of the heat generating body in the region) in the case where the heat generating body has a plurality of the heat generating members.

6. The heat generating body according to claim 4, wherein non-adhesive sections without the adhesive layer in the region are connectedly formed also on the mesh-like sheet while adjusting the width of the non-adhesive sections in a direction orthogonal to the longitudinal direction of the heat generating body.

7. The heat generating body according to claim 1, wherein the adhesive strength of the adhesive layer is 2.0 to 3.3 N/10.5 mm, and the shearing force is 6.0 to 8.5 kgf/29 mm.

8. The heat generating body according to claim 1, wherein the heat generating member is formed by holding a heat generating composition between a covering material formed of an air permeable material located on the mesh-like sheet side, and a base material formed of an air impermeable material located on the side opposite to the mesh-like sheet, and enclosing the heat generating composition between the covering material and the base material so as to form a seal part at the outer peripheral edges of the covering material and base material.

9. The heat generating body according to claim 1, wherein the contact surface side of the heat generating member is covered with a protective sheet.

10. The heat generating body according to claim 9, wherein the protective sheet is a non-woven fabric.

11. The heat generating body according to claim 9, wherein a resin film is provided on the heat generating member side of the protective sheet.

12. The heat generating body according to claim 9, wherein protrusions with a height of 0.3 to 2.0 mm are provided on the contact surface side of the protective sheet.

13. The heat generating body according to claim 1, wherein a release sheet is provided at a part or the whole of the adhesive layer.

14. The heat generating body according to claim 1, wherein a medicine is contained in a part of at least one member out of the surface of the heat generating member, the mesh-like sheet and the protective sheet which are components of the heat generating body.

15. The heat generating body according to claim 14, wherein a medicine is contained in the heat generating body by arranging a medicine layer adjacently to the heat generating body.

16. The heat generating body according to claim 1, wherein the mesh-like sheet is a non-woven fabric.

17. The heat generating body according to claim 1, wherein the mesh-like sheet is larger by 10% or more in the elongation percentage difference in the longitudinal direction of the heat generating body between the mesh-like sheet and the heat generating member provided at the heat generating body.

18. A heat generating body **characterized in that** mesh-like sheets are provided at both ends of the wide surface of the heat generating member having the wide surface, and an adhesive layer is provided on each mesh-like sheet.

## Amended claims

### Amended claims under Art. 19.1 PCT

**1.** Amended) A heat generating body **characterized in that**:
a heat generating member having a wide surface is provided on a mesh-like sheet larger in outer shape than the one-side wide surface of the heat generating member, and an adhesive layer is provided on the mesh-like sheet located at the outer peripheral part of the heat generating member;
the heat generating member is formed by holding a heat generating composition between a covering material formed of an air permeable material located on the mesh-like sheet side, and a base material formed of an air impermeable material located on the side opposite to the mesh-like sheet, and enclosing the heat generating composition between the covering material and the base material so as to form a seal part at the outer peripheral edges of the covering material and base material;
the adhesive layer is formed in a range of 10 to 80% of the surface area of a region, in which the heat generating member is located, on the mesh-like sheet (excluding the seal part); and
the adhesive layer is formed at the seal part of the heat generating member extending in an orthogonal direction to the longitudinal direction of the heat generating body.

**2.** Cancelled)

**3.** Cancelled)

**4.** Cancelled)

**5.** Amended) The heat generating body according to claim 1, wherein a region surrounded by the heat generating members located on the outermost peripheral edge is made the region (excluding the seal parts of the heat generating members located at both ends in the longitudinal direction of the heat generating body in the region) in the case where the heat generating body has a plurality of the heat generating members.

**6.** Amended) The heat generating body according to claim 1, wherein non-adhesive sections without the adhesive layer in the region are connectedly formed also on the mesh-like sheet while adjusting the width of the non-adhesive sections in a direction orthogonal to the longitudinal direction of the heat generating body.

**7.** The heat generating body according to claim 1, wherein the adhesive strength of the adhesive layer is 2.0 to 3.3 N/10.5 mm, and the shearing force is 6.0 to 8.5 kgf/29 mm.

**8.** Cancelled)

**9.** The heat generating body according to claim 1, wherein the contact surface side of the heat generating member is covered with a protective sheet.

**10.** The heat generating body according to claim 9, wherein the protective sheet is a non-woven fabric.

**11.** The heat generating body according to claim 9, wherein a resin film is provided on the heat generating member side of the protective sheet.

**12.** The heat generating body according to claim 9, wherein protrusions with a height of 0.3 to 2.0 mm are provided on the contact surface side of the protective sheet.

**13.** Amended) The heat generating body according to claim 1, wherein a release sheet is provided at a part or the whole of the adhesive layer, and the release sheet is provided with a folded part.

**14.** The heat generating body according to claim 1, wherein a medicine is contained in a part of at least one member out of the surface of the heat generating member, the mesh-like sheet and the protective sheet which are components of the heat generating body.

**15.** The heat generating body according to claim 14, wherein a medicine is contained in the heat generating body by arranging a medicine layer adjacently to the heat generating body.

**16.** The heat generating body according to claim 1, wherein the mesh-like sheet is a non-woven fabric.

**17.** The heat generating body according to claim 1, wherein the mesh-like sheet is larger by 10% or more in the elongation percentage difference in the longitudinal direction of the heat generating body between the mesh-like sheet and the heat generating member provided at the heat generating body.

**18.** Cancelled)

Statement under Art. 19.1 PCT
With respect to the heat generating body in claim 1, it is clarified that the adhesive layer is formed in a range of 10 to 80% of the surface area of a region, in which the heat generating member is located, on the mesh-like sheet and that the adhesive layer is formed at the seal part.

With respect to the holder of the chemical body warmer in the cited document 1 (Japanese Utility Model Application No. 65318/1986), the seal part of the "chemical body warmer" is not bonded to the "base fabric."

According to the present invention, an air permeability rate enough to obtain the sufficient sense of warmth as the heat generating body can be obtained, and the heat generating member can be firmly stuck to the mesh-like sheet so as not to fall off. Furthermore, heat generation for many hours at a stable temperature is attained.
